Europäisches Patentamt

(19) European Patent Office

Office européen des brevets

(11) Publication number: **0 109 281**
A1

## (12) EUROPEAN PATENT APPLICATION

(21) Application number: **83306897.6**

(22) Date of filing: **11.11.83**

(51) Int. Cl.³: **A 61 K 31/19**, A 61 K 31/215 // (A61K31/19, 31/165), (A61K31/215, 31/165)

(30) Priority: 15.11.82 US 441463
15.11.82 US 441466

(43) Date of publication of application: **23.05.84**
**Bulletin 84/21**

(84) Designated Contracting States: **BE CH DE FR GB IT LI NL SE**

(71) Applicant: **THE UPJOHN COMPANY, 301 Henrietta Street, Kalamazoo, Michigan 49001 (US)**

(72) Inventor: **Berkowitz, Marvin, The Upjohn Company 301 Henrietta Street, Kalamazoo Michigan 49001 (US)**

(74) Representative: **Perry, Robert Edward et al, GILL JENNINGS & EVERY 53-64 Chancery Lane, London WC2A 1HN (GB)**

(54) Compositions comprising flurbiprofen or ibuprofen.

(57) Pharmaceutical compositions comprise, as a first active ingredient, flurbiprofen or ibuprofen (or an ester or salt thereof), acetaminophen as a second active ingredient, and a carrier, for combined administration. For concomitant administration, a package includes separate compositions respectively comprising the first and second active ingredients. Use of such compositions, for combined or concomitant administration of the first and second active compounds, allows a reduction in the normal therapeutic dosage of acetaminophen.

0109281

## COMPOSITIONS COMPRISING FLURBIPROFEN OR IBUPROFEN

Flurbiprofen and ibuprofen are non-steroidal antiinflammatory drugs which are used in rheumatic and degenerative diseases of the joints and for reducing platelet adhesiveness. Ibuprofen has been used to reduce dental pain.

Acetaminophen is a known analgesic and anti-pyretic drug. It is used in a wide variety of arthritic and rheumatic conditions involving musculoskeletal pain, for headache, dysmenorrhea, myalgesia and neuralgias, and as an analgesic anti-pyretic in patients who are sensitive to aspirin. The usual adult oral dose is 0.3 to 1 g, administered three or four times a day.

The administration of acetaminophen as a supplemental analgesic in clinical studies of flurbiprofen administration is disclosed in European Journal of Rheumatology and Inflammation, vol. 3, no. 3, pp. 180-186 (1980) and Br., J. Clin. Pharm. 11, pp. 477-484 (1981). The administration of acetaminophen as a supplemental analgesic in clinical studies of ibuprofen administration is disclosed in Scand. J. Rheumatology 7: 113-117 (1978) and Annals of the Rheumatic Diseases, vol. XXVIII, pp. 513-517 (1969).

A pharmaceutical composition according to the invention comprises flurbiprofen or ibuprofen, or a $C_{1-8}$ alkyl ester or pharmaceutically acceptable salt thereof; acetaminophen; and a pharmaceutically acceptable carrier. For concomitant administration, a package comprises a first pharmaceutical composition comprising flurbiprofen or ibuprofen, or a $C_{1-8}$ alkyl ester or pharmaceutically acceptable salt thereof, and a pharmaceutically acceptable carrier, and a second composition comprising acetaminophen and a pharmaceutically acceptable carrier. Examples of esters are methyl, ethyl, isopropyl and octyl esters. Examples of salts are sodium, potassium and ammonium salts.

Administration of compositions of the invention allows acetaminophen to be used in an amount less then its usual therapeutic analgesic dose. The low amount of acetaminophen in the combination provides the advantage of reducing the gastric erosion or distress that can accompany the administration of a therapeutic amount of flurbiprofen.

The dose of flurbiprofen, its salts or esters, is the same dose known for treating conditions for which it is known to be useful. In general, from about 1 mg to about 5 mg per kilogram body weight is administered daily in single or divided oral dosage amount.

The dose of ibuprofen, its salts or esters, is the same dose known for teaching conditions for which it is known to be useful. In general, from about 5 mg to about 50 mg per kilogram body weight is administered daily in single or divided oral dosage amounts.

The amount of acetaminophen to be administered concomitantly is less than the therapeutic dose of 300 mg to 1 gm, preferably about 60% or less.

The acetaminophen and flurbiprofen or ibuprofen can be administered in the same dosage unit or can be prepared in separate dosage units and the dosage units administered at the same time. Different forms of dosage units can be used, i.e., a tablet of flurbiprofen or ibuprofen and a capsule of acetaminophen.

The compositions of the present invention are preferably presented for oral administration to humans or animals in unit dosage forms, such as tablets, capsules, pills, powders, granules, and oral syrups, solutions, or suspensions, and the like, containing suitable quantities of the combination of active ingredients.

For oral administration either solid or fluid unit dosage forms can be prepared.

Powders are prepared quite simply by comminuting the active ingredients to a suitably fine size and mixing with a similarly comminuted diluent. The diluent can be an edible carbohydrate material such as lactose or starch. Advantageously, a sweetening agent or sugar is present as well as a flavoring oil.

Capsules are produced by preparing a powder mixture and filling into formed gelatin sheaths. Advantageously, as an adjuvant to the filling operation, lubricant such as talc magnesium stearate, calcium stearate and the like is added to the powder mixture before the filling operation.

Soft gelatin capsules are prepared by machine encapsulation of a slurry of active ingredients with an acceptable vegetable oil, light liquid petrolatum or other inert oil or triglyceride.

Tablets are made by preparing a powder mixture, granulating or slugging, adding a lubricant and pressing into tablets. The powder mixture is prepared by mixing the active ingredients, suitably comminuted with a diluent or base such as starch lactose, kaolin, dicalcium phosphate and the like. The powder mixture can be granulated by wetting with a binder such as corn syrup, gelatin solution, methylcellulose solution or acacia mucilage and forcing through a screen. As an alternative to granulating, the powder mixture can be slugged, i.e., run through the tablet machine and the resulting imperfectly formed tablets broken into pieces (slugs). The slugs can be lubricated to prevent sticking to the tablet-forming dies by means of the addition of stearic acid, a stearate salt, talc or mineral oil. The lubricated mixture is then compressed into tablets.

Advantageously, the tablet can be provided with a protective coating consisting of a sealing coat or enteric coat of shellac, a coating of sugar and methylcellulose and a polish coating of carnauba wax.

Fluid unit dosage forms for oral administration such as syrups, elixirs and suspensions can be prepared wherein each teaspoonful of composition contains a predetermined amount of the active ingredients for administration. The water-soluble forms can be dissolved in an aqueous vehicle together with sugar, flavoring agents and preservatives to form a syrup. An elixir is prepared by using a hydroalcoholic vehicle with suitable sweeteners together with a flavoring agent. Suspensions can be prepared of the insoluble forms with a suitable vehicle with the aid of a suspending agent such as acacia, tragacanth, methylcellulose and the like.

The term "unit dosage form" as used in the specification and claims refers to physically discrete units suitable as unitary dosages for human and animal subjects, each unit containing a predetermined quantity of flurbiprofen or ibuprofen calculated to produce the desired therapeutic effect in association with acetaminophen and the required pharmaceutical diluent, carrier or vehicle. The specification for the novel unit dosage forms of this invention are dictated by and are directed dependent on (a) the unique characteristics of flurbiprofen or ibuprofen and the particular therapeutic effect to be achieved, and (b) the limitation inherent in the art of compounding

0109281

such an active material for therapeutic use in humans, as disclosed in this specification, these being features of the present invention. Examples of suitable unit dosages forms in accord with this invention are tablets, capsules, troches, powder packets, wafers, cachets, teaspoonfuls, tablespoonfuls, segregated multiples of any of the foregoing and other forms as herein described.

The following examples are illustrative of the present invention, but are not intented to be limiting.

Example 1    Hard Gelatin Capsules

One thousand two-piece hard gelatin capsules for oral use, each capsule containing 50 mg of flurbiprofen and 150 mg acetaminophen are prepared from the following types and amounts of ingredients:

| | |
|---|---|
| Flurbiprofen | 50 gm |
| Acetaminophen | 150 gm |
| Lactose | 100 gm |
| Corn starch | 20 gm |
| Talc | 20 gm |
| Magnesium stearate | 2 gm |

The flurbiprofen and acetaminophen (finely divided by means of an air micronizer) are added to the other finely powdered ingredients, mixed thoroughly and then encapsulated in the usual manner.

The foregoing capsules are useful for treating rheumatic diseases by the oral administration of one capsule four times a day.

Using the procedure above, capsules are similarly prepared containing acetaminophen in 100 and 75 mg amounts by substituting 100 and 75 gm of acetaminophen for the 150 gm used above.

Examples 2    Soft Gelatin Capsules

One-piece soft gelatin capsules for oral use, each containing 50 mg of flurbiprofen and 75 mg of acetaminophen (finely divided by means of an air mirconizer) are prepared by first suspending the compounds in 0.5 ml of corn oil to render the material capsulatable and then capsulating in the above manner.

The foregoing capsules are useful for treating rheumatic diseases by the oral administration of one capsule four times a day.

Example 3    Tablets

One thousand tablets, each containing 100 mg of flurbiprofen and 150 mg acetaminophen are prepared from the following types and amounts of ingredients:

| Flurbiprofen | 100 gm |
|---|---|
| Acetaminophen | 150 gm |
| Lactose | 75 gm |
| Corn starch | 50 gm |
| Magnesium stearate | 4 gm |
| Light liquid petrolatum | 5 gm |

The flurbiprofen and acetaminophen (finely divided by means of an air micronizer) are added to the other ingredients and then thoroughly mixed and slugged. The slugs are broken down by forcing then through a number sixteen screen. The resulting granules are then compressed into tablets, each tablet containing 100 mg of flurbiprofen and 150 mg of acetaminophen.

The foregoing tablets are useful for treating rheumatic diseases by the oral administration of one tablet four times a day.

Example 4    Oral Suspension

One thousand ml of an aqueous suspension for oral use, containing in each teaspoonful (5 ml) dose, 100 mg of flurbiprofen aluminum salt and 75 mg of acetaminophen is prepared from the following types and amounts of ingredients:

| Flurbiprofen, aluminum salt, micronized | 20 gm |
|---|---|
| Acetaminophen, micronized | 15 gm |
| Citric acid | 2 gm |
| Benzoic acid | 1 gm |
| Sucrose | 700 gm |
| Tragacanth | 5 gm |
| Lemon oil | 2 gm |
| Deionized water, q.s. | 1000 ml |

The citric acid, benzoic acid, sucrose, tragacanth and lemon oil are dispersed in sufficient water to make 850 ml of suspension. The acetaminophen and flurbiprofen aluminum salt (finely divided by means of an air micronizer) is stirred into the syrup until uniformly distributed. Sufficient water is added to make 1000 ml.

The composition so prepared is useful for treating rheumatic diseases at a dose of one tablespoonful (15 ml) four times a day.

Example 5    Hard Gelatin Capsules

One thousand two-piece hard gelatin capsules for oral use, each capsule containing 400 mg of ibuprofen and 150 mg of acetaminophen are

Example 5

Hard gelatin capsules are prepared, as in Example 1, but with the substitution of 400 g ibuprofen for the flurbiprofen ingredient. Each capsule contains 400 mg ibuprofen, and is useful for the same purpose as the capsules of Example 1.

Example 6

Soft gelatin capsules are prepared, as in Example 2, but with the substitution of ibuprofen for flurbiprofen, such that the capsules contain 200 mg ibuprofen each. They are useful for the same purposes as those of Example 2.

Example 7

Tablets are prepared, as in Example 3, but with the substitution of 800 g micronised ibuprofen for the flurbiprofen ingredient. The tablets contain 800 mg ibuprofen each, and are useful for the same purposes as the tablets of Example 3.

Example 8

An oral suspension is prepared, as in Example 4, but with the substitution of 40 g micronised aluminium ibuprofen for the flurbiprofen salt. The suspension contains 200 mg ibuprofen aluminium salt per 5 ml dosage, and is useful for the same purposes as the suspension of Example 4.

CLAIMS

1. A pharmaceutical composition which comprises flurbiprofen or ibuprofen, or a $C_{1-8}$ alkyl ester or pharmaceutically acceptable salt thereof; acetaminophen; and a pharmaceutically acceptable carrier.

2. A package which comprises a first pharmaceutical composition comprising flurbiprofen or ibuprofen, or a $C_{1-8}$ alkyl ester or pharmaceutically acceptable salt thereof, and a pharmaceutically acceptable carrier; and a second pharmaceutical composition comprising acetaminophen and a pharmaceutically acceptable carrier.

3. A composition or package according to claim 1 or claim 2, in which the or each composition is in the form of a tablet.

4. A composition or package according to claim 1 or claim 2, in which the or each composition is in the form of an encapsulated powder or liquid.

5. A package according to any of claims 2 to 4, for simultaneous, separate or sequential use in the management of pain.

European Patent
Office

**EUROPEAN SEARCH REPORT**

Application number

EP 83 30 6897

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 012 621 (MCNEILAB, INC.)<br>* Page 48, line 1 - page 53, line 2; claims 1-17 *<br>--- | 1-5 | A 61 K  31/19<br>A 61 K  31/215//<br>(A 61 K  31/19<br>A 61 K  31/165)<br>(A 61 K  31/215 |
| A | US-A-3 439 094 (JANE F. EMELE)<br>* Column 3, line 4 - column 4, line 3; claims 1-3 *<br>--- | 1-5 | A 61 K  31/165) |
| A,P | CHEMICAL ABSTRACTS, vol. 99, no. 5, August 1, 1983, page 32, no. 32896g, Columbus, Ohio, US<br>A.A. VAN KOLFSCHOTEN et al.: "Protection by paracetamol against various gastric irritants in the rat" & TOXICOL. APPL. PHARMACOL. 1983, 69(1), 37-42 * Abstract *<br>--- | 1-5 | |
| A,P | EP-A-0 085 544 (THE UPJOHN COMPANY)<br><br>----- | 1-5 | **TECHNICAL FIELDS SEARCHED (Int. Cl. 3)**<br><br>A 61 K |

The present search report has been drawn up for all claims

| Place of search | Date of completion of the search | Examiner |
|---|---|---|
| THE HAGUE | 14-02-1984 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document